(19)

Europäisches Patentamt
European Patent Office
Office européen des brevets

(11)  **EP 3 674 418 A1**

(12)  **EUROPEAN PATENT APPLICATION**

(43) Date of publication:
**01.07.2020  Bulletin 2020/27**

(21) Application number: **18382992.8**

(22) Date of filing: **26.12.2018**

(51) Int Cl.:
*C12Q 1/6841* (2018.01)   *G16B 5/00* (2019.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Life Length S.L.**
**28010 Madrid (ES)**

(72) Inventors:
• **Najarro Parra, María Pilar**
  **28010 MADRID (ES)**

• **Fernández Canivell, Luis**
  **28010 MADRID (ES)**
• **Esteban la Fuente, Laura**
  **28010 MADRID (ES)**
• **De Pedro Montejo, Nuria**
  **28010 MADRID (ES)**
• **Diez Zaera, María**
  **28010 MADRID (ES)**
• **Garcia Martínez, Jorge**
  **28010 MADRID (ES)**

(74) Representative: **Pons Ariño, Angel**
**Pons Patentes y Marcas Internacional, S.L.**
**Glorieta Rubén Dario 4**
**28010 Madrid (ES)**

(54)  **METHOD FOR MEASURING TELOMERE ASSOCIATED VARIABLES AND USES THEREOF FOR THE DIAGNOSIS AND/OR PROGNOSIS OF TELOMERIC-ASSOCIATED DISEASES**

(57)    The invention provided methods *in vitro* for diagnosis and/or prognosis of a clinical outcome in a subject with a telomere-associated disease comprising determining specific telomere associated variables (TAVs) of a test sample cell from the subject, wherein the value of the TAVs obtained for the test sample compared to a control sample is indicative of the telomerase-associated disease and/or the clinical outcome of the subject.

EP 3 674 418 A1

**Description**

The invention relates to methods *in vitro* for the determination of the specific Telomere Associated Variables (TAVs) values which can be correlated with: a measure of health; a risk of a pathological condition; a telomeric disease or drug responsiveness. Particularly, the present invention relates to methods *in vitro* to diagnose and/or assess telomere-associated diseases occurrence and/or severity. This invention falls within the field of molecular biology, biotechnology and medicine.

**BACKGROUND ART**

Telomeres, the tips of eukaryotic chromosomes, protect the chromosomes from nucleolytic degradation, end-to-end fusion, and recombination. Telomeres shorten as a consequence of normal cell division and critically short telomeres lead to cellular senescence or apoptosis. This process occurs because the DNA-replication machinery is incapable of fully replicating the ends of linear molecules, and, degradation and oxidative damage of nucleotides in DNA results. Critically short telomeres trigger loss of cell viability in tissues, which has been related to alteration of tissue function and loss of regenerative capabilities in ageing and ageing-related diseases. Hence, telomere length is an important biomarker for aging that can be used in the prognosis of ageing diseases. On the other hand, cancer cells prevent telomere shortening by activating telomere elongation mechanisms, which are important targets for cancer therapies. The former facts highlight the importance of accurately measuring telomere length in cancer and age-related disease studies.

A rich body of epidemiological and clinical studies in humans in the past decade has linked short telomere length to high risks of cancer and aging-related disease and all-cause mortality. Genetic, environment, lifestyle, and behavioral factors collectively impact telomere length. Therefore, telomere length has become an index for overall health, disease, and mortality risk. While average telomere length was measured in almost all the clinical studies published and have demonstrated utility in stratifying patient disease and mortality risk, other works has demonstrated that the quantity of short telomeres, and not average telomere length, is one of the critical factors for cell viability and chromosome stability (Hemann, M.T., et al. Cell, 2001. 107(1) 67-77). Therefore, changes in the percentage of short telomere abundance are expected to be a more sensitive measurement of the effects of lifestyle and pharmacological or other interventions on telomeres (Vera E., et al. Cell Rep. 2012 Oct 25;2(4):732-7). In this sense, the telomeric variables such as, the analysis of the telomere length and the percentage of short telomere abundance in a sample, have been used as biomarkers to allow for improvements in risk assessments of diverse health outcomes. However, the utility of the measure depends on valid and reliable techniques to quantify these telomeric variables.

Various methods have been developed for the measurement of telomere length and the percentage of short telomere abundance in genomic DNA, including Southern blotting, qPCR, Q-FISH, flow-FISH and HT Q-FISH. All of these methods can be used in a clinical setting to monitor health status and permit physicians to prescribe prophylactic or therapeutic intervention tailored to the needs of the individual patient.

The qPCR technique uses primers for the telomere region and a single copy gene (may be on the same chromosome or on a different chromosome). PCR-based techniques have become a popular method for estimating telomere length due to relatively low cost, amenability for high-throughput testing, and relative ease of investigators' access to the necessary equipment used in the assay. While the PCR-based techniques are well suited for large epidemiological studies, the results are difficult to compare between studies. This limitation is due to differences in the DNA quality based on the method used for genomic DNA extraction, as well as differences in sample fixation methods in the case of fixed and paraffin-embedded tissue samples and by their relatively high level of variation among replicate estimates.

Flow-FISH as its name suggests, combines flow cytometry methodology with the hybridization of a pantelomeric (binds to all telomeres) probe to cells in a suspension (rather than hybridizing to cells fixed to slides, as is done for metaphase and interphase Q-FISH). Flow-FISH methodology typically uses the same telomeric $(CCCTAA)_3$ PNA probe used in other Q-FISH approaches to quantify the mean amount of fluorescence present in cells. This value is then used to provide an average telomere length for the cell population being evaluated. A strength of this approach is that it has the capability to sort cells into subpopulations based upon size, granulation, and/or antibody labeling. As a result of this potential, flow-FISH has been widely used for determining mean telomere length in hematopoietic cell subtypes. Flow-FISH is also the first of the telomere assays to be used as a clinical diagnostic tool, with the method being used to assist with the recognition of patients having dyskeratosis congenita-a condition that is associated with shortened telomere lengths. Flow-FISH has limitations that may compromise suitability for use in research/clinical studies. Specifically, unfixed cells can be challenging to process (fragility, clumping, etc.), but the technique is sensitive to fixatives used to preserve cells, with the reliability of the measures reflecting these technical parameters. Also, the PNA probe used in flow-FISH has been shown to demonstrate nonspecific binding to cytoplasmic structures. Thus, it been suggested that isolated nuclei, rather than intact cells, may be optimal for assessment with this methodology. Due to the above noted technical issues, flow-FISH is not readily adaptable for use in a wide range of cell types, with its application being primarily for use with fresh blood samples. Also, like many of the other techniques, this method provides only a mean value of telomere intensity, and provides no information regarding individual telomeres or a subset of shortened telomeres.

Q-FISH methodologies use a probe specific for the telomeric region of metaphase-spread cells to estimate length based on histograms of telomere signal intensities which represent the length of individual telomeres. While the probe tends to be specific for the telomeric region, it is possible that the probe could bind to a portion of the juxtaposed region (especially the degenerate repeat region). Limitations of this method are that live cells are needed, costs are high, and throughput is low. Thus, this procedure is not well suited for large, epidemiological studies.

To overcome some of the limitations of Q-FISH, adaptations of this procedure have been developed, with these adaptations involving the use of interphase cells rather than metaphase chromosomes. Interphase Q-FISH is amenable for assessing telomere lengths in nuclei from multiple specimen types (blood cells; formalin-fixed; paraffin embedded tissues; frozen tissues). A clear advantage for using interphase Q-FISH methodology is that it allows to concurrently collect information regarding telomere length and histological information, having the potential to be combined with immunostaining techniques to localize specific cells of interest (sometimes referred to as "telomapping," (Vera E., et al. Cell Rep. 2012 Oct 25;2(4):732-7). A disadvantage of the interphase Q-FISH method is that it does not allow for the recognition of specific telomeres, and does not allow for the detection of telomere-free ends. Also, the data is typically presented as a mean value, since the overlaying of the 92 telomeres can prohibit one from unequivocally recognizing each individual telomere. However, interphase Q-FISH is less labor intensive than metaphase Q-FISH, with recent adaptations of this technique, called high-throughput (HT) Q-FISH are amenable to automation and for use in larger epidemiological studies.

Although up to now, the main telomeric variables used as useful biomarkers for the diagnosis and/or prognosis of cancer and aging-related diseases are the telomere length and the percentage of short telomere abundance in genomic DNA, which can be determined by the methods mentioned above. There remains a need for a more reliable, accurate and efficient telomeric variables for use as biomarkers for the diagnosis and/or prognosis of cancer and aging-related diseases, which can be analysed by a rapid, reliable, accurate, and efficient high-throughput method.

## SUMMARY OF THE INVENTION

The present invention provides an improved *in vitro* method for measuring specific TAVs. The *in vitro* methods of the invention can be done rapidly and provide increased sensitivity, efficiency, reliability, and accuracy. Moreover, these *in vitro* methods are amenable to automation and high through-put formats and provide, in some embodiments, a means to measure the TAVs of an individual chromosome, to compare interchromosomal variance in the specific TAVs, and to measure specific TAVs of a specific cell population within a mixture of cells. The present invention provides numerous advantages over all other methods of telomere length measurement:

- It is unique as it allows the determination of a distinctive profile of TAVs and their Principal Component Analysis treatment.
- Unlike flow-FISH, TRF, STELA and qPCR it makes it possible to detect and register the full distribution of the telomere length in a biological sample and evaluate differences in the ratios and proportions of short and the long telomere populations.
- Unlike qPCR that reports relative telomere length this method uses an internal standard curve that result in the exact determination of absolute telomere lengths in base pairs.
- Unlike TRF and STELA it is a scalable, industrially applied methodology and can be automatized.
- Unlike flow-FISH it is not limited to measure the average telomere signal of a cell but of each telomere individually.
- Moreover, the method can be applied to any cell type.

The data presented herein shows that the specific TAVs analysed within cells and group of cells, have a defined distribution that is specific and that the shape of this distribution determines disease status of such cells or group of cells. This is due to the specific TAVs variation associated with both normal and abnormal cell division. The length of telomeres and their ability to protect the genome are modified in tumor cells such that the particular variables associated with the distribution of the TAVs can be determined and analysed in a given biological sample allowing the application of these values to determine the health status of the subjects. Thus, based on the present findings, it is now possible to reliably examine and quantify the presence or absence of individual specific TAVs mentioned herein in interphase nuclei and to monitor them over time. Such new avenues will impact on cancer biology, genetics and diagnostic and/or prognosis innovations in medicine.

The method of the present invention used high resolution fluorescence microscopy and imaging to elucidate the individual length of telomeres and chromosomes in the interphase nuclei of analysed cells. It has been found that telomeres from test cells have a defined distribution, and that this distribution differs from their normal counterparts.

The results obtained in the present studies show that the specific TAVs of the invention analysed in a cell or in a group of cells are correlated with high accuracy with a measure of health, a risk of a pathological condition, a telomeric disease or responsiveness to a drug.

Also, disclosed herein are methods and systems for determining with a high accuracy a measure of health; a risk of a pathological condition; a telomeric disease or drug responsiveness based on the measurement of specific TAVs comprising at least one of the list selected from: absolute deviation of the median (MAD 12) of the telomeric length, percentage of long telomeres, percentage of cells having short telomeres or any combinations thereof. The method disclosed herein allow for; 1) distinction of normal and tumor cells, 2) identification of patient subgroups that will allow for new treatment designs, 3) identification of patients who will recur and therefore should obtain different treatments, 4) treatment monitoring, and 5) personalized medical management of patients (not one treatment for all, but a treatment specifically adapted to each patient).

In one aspect, the present invention provides a method *in vitro* for measuring TAVs, hereinafter first method *in vitro* of the invention, in an isolated biological sample of a subject, wherein the method comprises the following steps:

a) assaying, in the isolated biological sample, at least one of the TAVs selecting from the list consisting of: MAD I2 of the telomeric length, percentage of long telomeres, percentage of cells having short telomeres, or any combinations thereof;

b) comparing the TAVs obtained in step a) with the TAVs obtained in a reference sample, and

c) identifying a significant difference between the TAVs comparison of step b), wherein a significant difference is indicative of: a measure of health; a risk of a pathological condition; a telomeric disease or drug responsiveness.

In another preferred embodiment of the first method *in vitro* of the invention, the TVAs are determined, for example, by using standard hybridization techniques, such as fluorescence *in situ* hybridization (FISH), Quantitative Fluorescent *in situ* hybridization (Q-FISH), or High Throughput Quantitative Fluorescent *in situ* hybridization (HT Q-FISH). In a more preferred embodiment, the TVAs are determined by HT Q-FISH.

The term HT-Q-FISH as used herein refers to any technique that allows the processing of tens or hundreds of samples in a high-capacity multiwell plate, using high resolution microscopy. The image data obtained by the high-resolution microscopy can include the intensities of one or more colours at pixels that comprise an image of a nucleus or nuclei. The image data can also be grey level image data of a nucleus that has been appropriately stained to highlight telomeres and/or chromosomes. Several images, preferably at least 75 are obtained corresponding to slices along a particular axis.

To obtain an image of telomeres stains with different fluorescent dyes (fluorophores) are performed. As used herein, "fluorescent dyes" or "fluorochrome" refers to a particular fluorescent dye molecule or conjugated moiety, suitable for use as labels in the present method. These fluorochromes can be selected from any of the many dyes suitable for use in imaging applications. A large number of dyes are commercially available from a variety of sources that provide great flexibility in selecting a set of dyes having the desired spectral properties. Examples of fluorophores include, but are not limited to, 4-acetamido-4'-isothiocyanatostilbene-2,2' disulfonic acid; acridine and derivatives such as acridine, acridine orange, acridine yellow, acridine red, and acridine isothiocyanate; cyanine and derivatives such as cyanosine, Cy3, Cy5, Cy5.5, and Cy7; 4',6-diaminidino-2-phenylindole (DAPI); 5',5"-dibromopyrogallol-sulfonephthalein (Bromopyrogallol Red); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6-dichlorotriazin-2-yl)aminofluorescein (DTAF), pyrene and derivatives such as pyrene, pyrene butyrate and succinimidyl 1-pyrene butyrate; Reactive Red 4 (Cibacron™ Brilliant Red 3B-A); rhodamine and derivatives such as 6-carboxy-X-rhodamine (ROX), 6-carboxyrhodamine (R6G), tetramethyl rhodamine, and tetramethyl rhodamine isothiocyanate (TRITC); riboflavin; rosolic acid and terbium chelate derivatives; xanthene; Alexa-Fluor dyes (e.g., Alexa Fluor 350, Alexa Fluor 430, Alexa Fluor 488, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, Alexa Fluor 750), Pacific Blue, Pacific Orange, Cascade Blue, Cascade Yellow; Quantum Dot dyes (Quantum Dot Corporation); Dylight dyes from Pierce (Rockford, III.), including Dylight 800, Dylight 680, Dylight 649, Dylight 633, Dylight 549, Dylight 488, Dylight 405; or combinations thereof. Other fluorophores or combinations thereof known to those skilled in the art may also be used.

A standard curve is built using the intensity data from an isolated biological sample of known telomere length. The standard curve will include immortalized cell lines whose median telomere length as determined by HT-QFISH are $\geq 2,000$ base pairs and $\leq 40,000$ base pairs. In this standard curve each of the telomere signals of measurable intensities obtained from the isolated test sample is interpolated into the standard curve and an absolute telomere length value is given to each of the telomere spots detected. TAVs are calculated involving the intensity of each individual telomere.

The terms "Telomeric Associated Variables", "TAVs", "Telomeric Related Variables" and/or "TRVs" as used herein refer to the individual measurements of telomeres and their distribution during any phase of a cell cycle and includes such parameters as telomere length, absolute telomere size measured as base pairs units, percentage of telomeres below or above a certain size, differences between longest and shortest telomeres, percentages of cells in a cell group having a particular telomere median length, MAD I2 of the telomeric length, etc. These terms also refer to the combination of several TAVs.

In a particular embodiment of the first method *in vitro* of the invention, the MAD I2 of the telomeric length in the isolated biological sample $Mt$ is determined by the following formula:

$$MAD = median(|m_i - \tilde{m}|)$$

wherein

m is the set of fluorescence intensities corresponding to the telomeres in the sample $M_t$ and $\tilde{m}$ is the median of m, $m = \{t_1, t_2, ..., t_n\}$;
$M_t = \{T_1, T_2, ..., T_n\}$ is the complete set of telomeres measured in the isolate biological sample; and
$t_i (\{t_1, t_2, ..., t_n\})$ is the fluorescence intensity of the telomere $T_i (\{T_1, T_2, ..., T_n\})$.

In another particular embodiment of the first method *in vitro* of the invention, the percentage of long telomeres $TL_u$, in the isolated biological sample $M_t$, is determined by the following formula:

$$TL_u = 100 - TS_u$$

wherein the percentage of short telomeres $TS_u$ in the isolated biological sample $M_t$ for a threshold length $u$ is determined as:

$$TS_u = 100 * \frac{\sum_{i=1}^{n}[t_i < u]}{n}$$

wherein

$M_t = \{T_1, T_2 ... T_n\}$ is the complete set of the telomeres measured in the isolated biological sample $M_t$,
$n$ is the number of telomeres in $M_t$, and
$t_i (\{t_1, t_2, ..., t_n\})$ is the telomeric length of the telomere $T_i (\{T_1, T_2, ..., T_n\})$.

Moreover, the threshold length $u$ ranges from 500 to 40,000 pb in increments of 100 pb.

In another particular embodiment of the first method *in vitro* of the invention, the percentage of cells having short telomeres ($CS_u$) in the isolated biological sample M for a threshold length $u$ is determined as:

$$CS_u = 100 * \frac{\sum_{r=1}^{n}[c_r < u]}{n}$$

wherein

$c_r$ is the length of $C_r \in M_c$ calculated as: $c_r = avg(t_{ri})$;
$n$ is the number of cells in $M_c$,
$t_{ri}$ is the telomeric length of the telomere $Tr_i$;
$K_r = \{T_{r1}, T_{r2}, ..., T_{rm}\}$ is the set of telomeres analyzed in a cell $C_r$; and
$M_c = \{C_{r1}, C_{r2}, ..., C_{rn}\}$ is the complete set of cells in the isolated biological sample M.

Moreover, the threshold length $u$ ranges from 500 to 40,000 pb in increments of 100 pb.

In a preferred embodiment of the first method *in vitro* of the invention, the step a) comprising the analysis of the three TAVs in combination: TAVs: MAD 12, percentage of long telomeres and percentage of cells having short telomeres.

In another preferred embodiment of the first method *in vitro* of the invention, further comprises in step a) the analysis of at least one TAV selected from the list consisting of: the differences in base pair units between percentile values 1th and 99th of the telomere distribution of the sample (P1-99), the difference in base pairs units between percentile

values 25th and 75th of the telomere distribution of the sample (P75-25), also known as interquartile (Q3-Q1), the nuclear size distribution of the cells in the sample, or any combinations thereof.

The term "telomere length" as used herein refers to the absolute size of telomeres as determined by interpolation onto the standard curve of the *in vitro* method of the invention expressed in base pairs. For example, telomeres with a length in base pairs (x-axis) ranging from 500 to 3,000 bp are classified as very short, with a length ranging from >3,000 to 5,000 bp as short, with a length ranging from >5,000 to 10,000 bp as mid-sized, with length from >10,000 to 15,000 bp as large and with lengths >15,000 bp as very large.

In a preferred embodiment of the first method *in vitro* of the invention, the long telomeres are preferably telomeres having at least 13,000 bp and more preferably more than 15,000 bp. In another preferred embodiment of the first method *in vitro* of the invention, the short telomeres are preferably telomeres having less than 5,000 bp.

In a more preferred embodiment of the first method *in vitro* of the invention, the cells having short telomeres are preferably, cells having an average telomere length below 5,000 bp.

In another preferred embodiment, the first method *in vitro* of the invention, further comprises an additional step wherein the measurement of the telomerase activity is performed in the isolated biological sample.

In another preferred embodiment of the first method *in vitro* of the invention, the telomerase activity is measurement by Q-TRAP.

The term "biological sample" as used herein, refers to a sample isolated from a subject. Examples of biological samples include but are not limited to cells, tissues and/or biological fluids of an organism obtained by means of any method known by a person skilled in the art. In a particular embodiment, the biological sample is a biological fluid. In a more particular embodiment the biological fluid is selected from the list consisting of plasma, serum, blood, lymphatic fluid, cerebrospinal fluid, synovial fluid, urine, saliva, mucous, phlegm and sputum. In one embodiment, the biological sample is selected from the group consisting of blood, serum, and plasma. In another embodiment, the isolated biological sample is a human sample. The isolated biological sample of the present invention may be collected by any suitable method. The biological sample may be used immediately or may be stored for later use. Any suitable storage method known in the art may be used to store the biological sample; for example the sample may be frozen at about -20° C to about - 196° C.

As used herein, the term "cell" includes more than one cell or a plurality of cells or portions of cells. An advantage of the method *in vitro* of the present invention is that any single cell suspension can be used. As used herein, the term "test cell" or "test sample" is a cell or sample from a subject that is suspected of having a cell-proliferative disorder such as cancer or a telomeric-associated disease. In such an embodiment, the test cell includes, but is not limited to, a cancer cell. The term "control cell" or "control sample" is a suitable comparator cell, e.g. a cell that is an age matched control, and preferably a disease-free cell, or a cell.

As used herein, the terms "subject" or "patient" refers to any animal, preferably a mammal and includes, but is not limited to, domestic and farm animals, primates, and humans, for example, human beings, non-human primates, cows, horses, pigs, sheep, goats, dogs, cats, or rodents like rats and mice. In a preferred embodiment, the subject is a human being of any age, sex or race. Thus, adult and newborn subjects, as well as fetuses, whether male or female, are intended to be covered.

In another preferred embodiment of the method *in vitro* of the invention, the step b) comprising the comparison of the value for the TAVs obtained step a) in the isolated biological test sample, with the value for the TAVs obtained in the reference or control sample.

In another preferred embodiment of the method *in vitro* of the present invention, the step c) comprising to identify a significant difference between the comparison of previous step b).

In another particular embodiment of the method *in vitro* of the invention, wherein there is a significant difference between the value for the TAVs obtained in the isolated test sample versus value for the TAVs obtained in the isolated control sample, is indicative of: a measure of health; a risk of a pathological condition; a telomeric disease or responsiveness to a drug.

The terms "reference sample" or "control sample" as used herein refer to an isolated biological sample(s) from a subject(s) that is considered a control. A reference sample could be, for example, an isolated biological sample from healthy subjects or from non-telomeric diseases subjects.

The term "control" as used herein means any tissue, biological sample or cell sample from one or more subjects not having telomeric disease (e.g. control subjects) or also having earlier stage non-telomeric disease, such as an age matched control or can be a reference value derived from a sample from a control subject or group of control subjects.

The term "reference value" as used herein relates to a predetermined criterion used as a reference for evaluating the values or data obtained from the samples collected from a subject. Particularly, the reference value or reference level is a suitable comparator such as a percentage, any value having significant difference regarding test sample, threshold or cut off value, for example corresponding to the TAVs mentioned in the present invention, which is associated with a telomeric-disease. The reference value or reference level can be an absolute value, a relative value, a value that has an upper or a lower limit, a range of values, an average value, a median value, a mean value, or a value as compared to a particular control or baseline value. In embodiments where the severity of the telomeric-disease is being compared, the reference value can be a disease reference value for example mild telomeric-disease reference value, moderate telomeric-disease reference value or severe telomeric-disease reference value, wherein a value such as a threshold value is determined for a population of subjects having similar disease e.g. mild telomeric-disease, moderate telomeric-disease or severe telomeric-disease. The reference value can be a value arising from population studies, theoretical models, or the characterization of control cells. The reference value can be based on a large number of samples, such as from population of subjects of the chronological age matched group, or based on a pool of samples including or excluding the sample to be tested.

In particular, the value of the TAVs, as determined using the *in vitro* method of the present invention, determine, as mentioned above, the diagnostic, prognostic or response to treatment of a disease. Therefore, in an embodiment of the present invention, the *in vitro* method to determine the presence of abnormal values of the TAVs in a test sample is indicative of cancer and/or telomeric disease.

The method *in vitro* of the invention can be performed rapidly and provide increased sensitivity, efficiency, reliability and accuracy. Moreover, this method for measurement the TAVs mentioned herein can be employed in a high through-put and/or automated process format. These TAVs measurement methods can be used for diagnostic, prognostic, and research applications. Such applications include, but are not limited to: (i) determination of the proliferative lifespan of cells; (ii) identification and analysis of the effectiveness of agents capable of extending, maintaining, or reducing telomere length; (iii) diagnosis of disease or medical conditions characterized by a different telomere length in a patient relative to an individual not having the disease or particular medical condition; (iv) prognosis of disease or medical conditions as correlated to telomere length; (iv) monitoring and evaluating the efficacy of a therapy specific for the telomeric-associated disease; and (v) screening for drug candidates affecting TAVs. In general, measurement of the specific TAVs disclosed herein provides a powerful means to assess and monitor cellular lifespan for a variety of useful purposes.

In general, conditions associated with telomere length have typically been derived using a measure of average telomere length. However, the present invention discloses the use of the MAD I2 of the telomeric length, the percentage of long telomeres and the percentage of cells having short telomeres, as TAVs which are useful since are more sensitive and potentially more accurate predictors of clinically meaningful outcomes (e.g. disease or mortality risk) than the measurement of average telomere length or the rate of change in average telomere length.

TAVs, as determined by the *in vitro* method of the present invention, show a degree of correlation that suggests that the predictive value of these variables may be potentiated by using a Principal Component Analysis (PCA). Accordingly, performing PCA with each block or set of variables, such as MAD-I2, short telomeres within each cell, cells with short telomeres and percentiles, can be used to discern particular conditions. Thus, if the test biological sample has a percentage of long telomeres, as measured by the accurate and precise *in vitro* method of the invention, that is significantly different from the percentage of long telomeres in the reference biological sample, then the test biological sample will be more prone to disease or mortality risk compared to biological samples with a non-significantly different percentage of long telomeres. Quantitative assessment of the percentage of long telomeres will have significant diagnostic utility in the health monitoring and also in disease diagnostics, prognosis, and companion diagnostics. In the same sense, a test biological sample with a value of average abundance of long telomeres significantly different than the average abundance of long telomeres compared to a reference biological sample (age-matched reference population of normal individuals) have an increased risk of morbidity or mortality. Such a person (test biological sample) would be motivated to change their behaviour towards a better lifestyle and increase the abundance of long telomeres, while an individual (test biological sample) with a greater average abundance of long telomeres than the average abundance of long telomeres in the reference population, would know that their cellular health is likely better than average, and hence would be motivated to at least to maintain, if not further improve, their lifestyle habits to maintain their good health. Moreover, as mentioned above, the *in vitro* method disclosed herein, for the determination of the TAVs of the invention, can be also used to monitor the effectiveness of a specific treatment, preferably the effectiveness of cancer chemotherapeutics during treatment.

As it is mentioned above, the method *in vitro* of the invention, using the TAVs mentioned herein, determined from isolated biological samples from subjects, can be correlated with measures of health. Of particular interest are measures of health involving perceived stress. Telomere shortening can be accelerated by genetic and environmental factors, including multiple forms of stress such as oxidative damage, psychological stress, post-traumatic stress disorder, biochemical stressors, chronic inflammation and viral infections. The most frequently studied diseases and conditions include arthritis, back pain, cancer, cardiovascular disease, chronic obstructive pulmonary disease, depression, diabetes, gastro-intestinal disease, migraine headache, HIV/aids, hypertension, irritable bowel syndrome, kidney disease, low back pain, multiple sclerosis, musculoskeletal conditions, neuromuscular conditions, osteoarthritis, psychiatric diagnoses, rheumatoid arthritis, sleep disorders, spinal injuries, stroke, substance abuse, surgical procedures, transplantation and trauma.

In a particular aspect of the *in vitro* method of the invention, the isolated biological sample from the subjects, are collected over time and measurements of TAVs are determined from the samples. Appropriate time periods for collection of a plurality of samples include, but are not limited to, 1 month, 3 months, 6 months, 1 year, 2 years, 5 years and 10 years (for example, the time between the earliest and the last sample can be about these time periods). The *in vitro* method of the invention allows for monitoring of patient efforts to improve their general health status and/or to monitor their health status and/or disease risk. Since short telomeres trigger cell death, a finding that the percentage of short telomere length is lowered or maintained with time within an individual indicates a health improvement, while increase of percentage of short telomeres overtime represents a decrease or worsening in health.

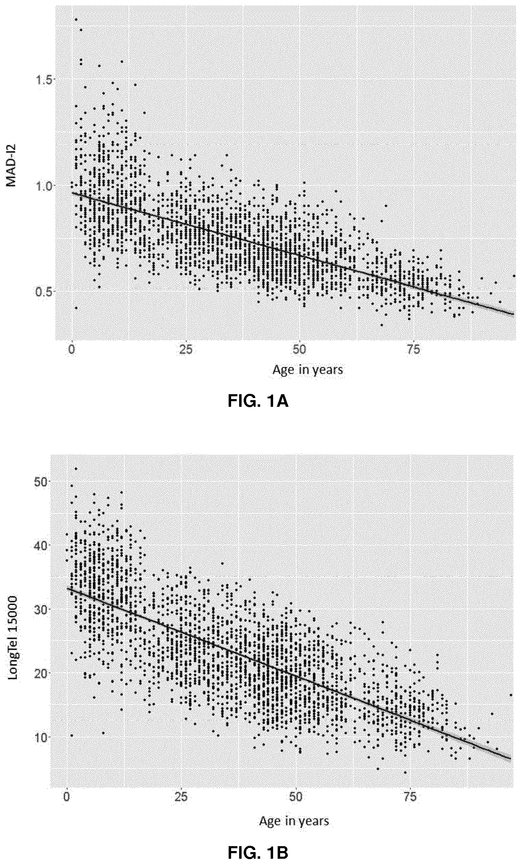

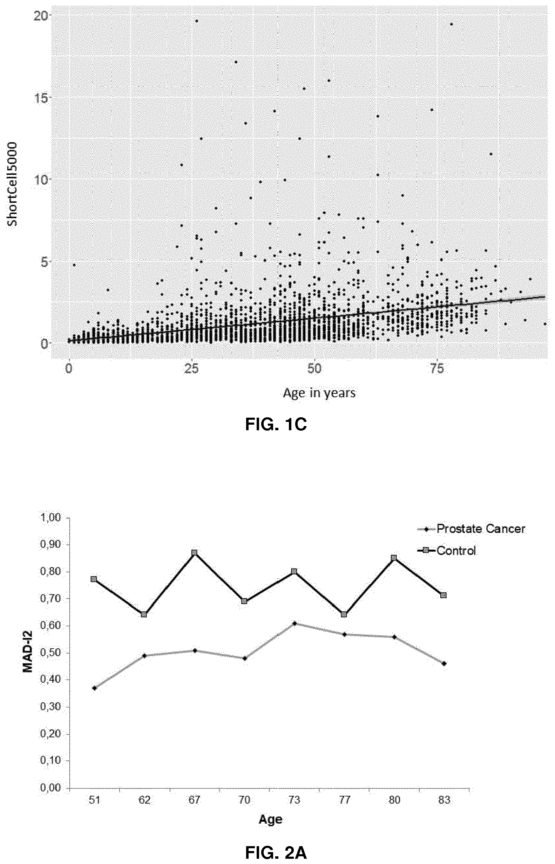

**Fig. 1A** shows that MAD-I2 diminished with age. In this sense, if MAD-I2 is increased or maintained overtime in an isolated biological sample from a subject, this is indicative of an improvement in the health status of this subject. Moreover, if MAD-I2 is decreased overtime in an isolated biological sample from a subject, this is indicative of a worsening in the health status of this subject. In the same sense, the percentage of long telomeres in an isolated biological sample diminished with age **(Fig. 1B)**. The maintenance of the percentage of long telomeres contributes to cellular health. Thus, if the percentage of long telomeres, preferably having at least 15,000 pb, is increased or maintained overtime in an isolated biological sample from a subject, this is indicative of an improvement in the health status of this subject. Moreover, if the percentage of long telomeres, preferably having at least 15,000 pb is decreased overtime in an isolated biological sample from a subject, this is indicative of a worsening in the health status of this subject. Additionally, the percentage of cells having short telomeres, preferably having less than 5,000 pb, increases with age **(Fig. 1C)**. Thus, if the percentage of cells having short telomeres, preferably having less than 5,000 pb, is decreased or maintained overtime in an isolated biological sample from a subject, this is indicative of an improvement in the health status of this subject. Moreover, if the percentage of cells having short telomeres, preferably having less than 5,000 pb, is increased overtime in an isolated biological sample from a subject, this is indicative of a worsening in the health status of this subject.

In one aspect of the *in vitro* method of the invention, the pathological condition is a telomeric-associated disease. Telomeric-associated diseases are disorders associated with an abnormal or premature shortening of telomeres, which can, for example, additionally result from defects in telomerase activity. These diseases may be inherited and include certain forms of congenital aplastic anemia, certain inherited diseases of the skin and the lungs, such as dyskeratosis congenita, idiopathic interstitial pneumonias and idiopathic pulmonary fibrosis. Additionally, telomeric-associated diseases also comprise, and are not limited to, cardiovascular disease, diabetes, cancer, liver fibrosis, and depression. Moreover, in another aspect of the *in vitro* method of the invention, the TAVs mentioned herein can be used as indicator of prognosis, disease progression and treatment outcome.

In one aspect of the *in vitro* method of the invention, both the presence and the progress of telomeric-associated diseases may be determined using isolated biological samples as mentioned above.

The term "cancer", as used herein, refers to a disease characterized by uncontrolled cell division (or by an increase of survival or apoptosis resistance) and by the ability of such cells to invade other neighbouring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. Biological processes known to be related to cancer include angiogenesis, immune cell infiltration, cell migration and metastasis. As used herein, the term cancer includes, but is not limited to, the following types of cancer: breast cancer; biliary tract cancer; bladder cancer; brain cancer including glioblastomas and medulloblastomas; cervical cancer; choriocarcinoma; colon cancer; endometrial cancer; esophageal cancer; gastric cancer; hematological neoplasms including acute lymphocytic and myelogenous leukemia; T-cell acute lymphoblastic leukemia/lymphoma; hairy cell leukemia; chronic myelogenous leukemia, multiple myeloma; AIDS-associated leukemias and adult T-cell leukemia/lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Merkel cell carcinoma, Kaposi's sarcoma, basal cell carcinoma, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor.

The term "metastatic progression", as used herein, refers to the process through which a tumor spreads to body tissues different than the primary site of tumor origin.

In another aspect, the *in vitro* method of the present invention is useful in monitoring effectiveness of therapeutics or in screening for drug candidates affecting telomere length and/or telomerase activity. The ability to monitor telomere characteristics can provide a window for examining the effectiveness of particular therapies and pharmacological agents. The drug responsiveness of a disease state to a particular therapy in an individual may be determined by the *in vitro* method of the present disclosure, wherein shorter telomere length correlates with better drug efficacy. For example, the present disclosure also relate to the monitoring of the effectiveness of cancer therapy since the proliferative potential of cells is related to the maintenance of telomere integrity.

The term "treatment", as used herein, refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. In various aspects, the term covers any treatment of a subject, including a mammal (e.g., a human), and includes: (i) preventing the disease from occurring in a subject that can be predisposed to the disease but has not yet been diagnosed as having it; (ii) inhibiting the disease, i.e., arresting its development; or (iii) relieving the disease, i.e., causing regression of the disease.

As used herein, the term "prevent" or "preventing" refers to precluding, averting, obviating, forestalling, stopping, or hindering something from happening, especially by advance action. It is understood that where reduce, inhibit or prevent are used herein, unless specifically indicated otherwise, the use of the other two words is also expressly disclosed.

As used herein, the term "prognosis" refers to an expected course of clinical disease. The prognosis provides an indication of disease progression and includes for example, an indication of likelihood of recurrence, metastasis, death due to disease, tumor subtype or tumor type. The prognosis can comprise a good prognosis which corresponds to a good clinical outcome relative to the spectrum of possible clinical outcomes for the specific cancer or syndrome, and a poor prognosis, which corresponds to a poor clinical outcome relative to the spectrum of possible clinical outcomes for the specific cancer or syndrome. As used herein, "good prognosis" means a probable course of disease or disease outcome that has increased quality-adjusted life-year (QALY), reduced morbidity and/or reduced mortality compared to the average for the disease or condition. As used herein, "poor prognosis" means a probable course of disease or disease outcome that has increased morbidity and/or increased mortality compared to the average for the disease or condition.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

**BRIEF DESCRIPTION OF THE DRAWINGS**

FIG. 1. Normograms for the TAVs of the invention. All the subjects from zero to 93 years of age were analyzed following HT-Q-FISH to obtain the TAVs of the invention MAD-I2 (**A**), percentage of long telomeres (LongTel15000) (**B**) and percentage of cells having short telomeres (ShortCell 5000 (**C**). In the x axis the individual age in years is correlated to the TAVs values in the y axis.

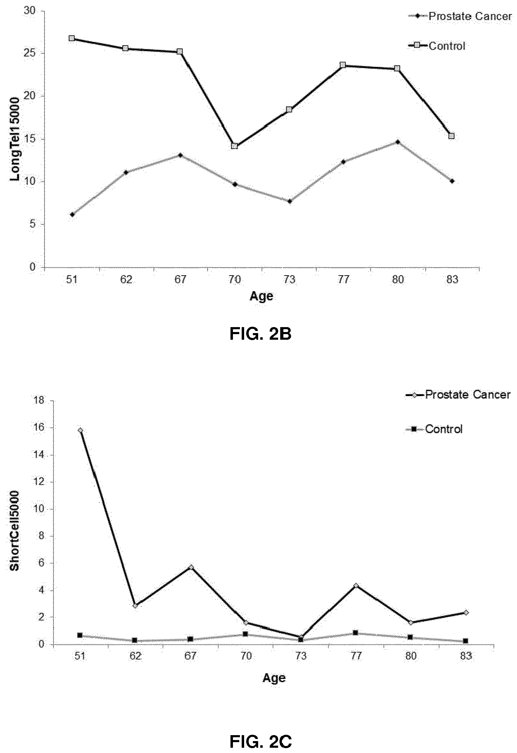

FIG. 2. The TAVs of the invention, such as (**A**) MAD-I2, (**B**) long telomeres > 15, 000 pb (LongTel15000) and (**C**) cells having short telomeres < 5,000 pb (ShortCell 5000), analyzed in biological sample from subjects having prostate cancer (positive biopsy) and from healthy control subject (negative biopsy) across the different age-ranges. Diamond shaped marked line represents patients diagnosed with prostate cancer. Square shaped marked line represents healthy controls matched by age.

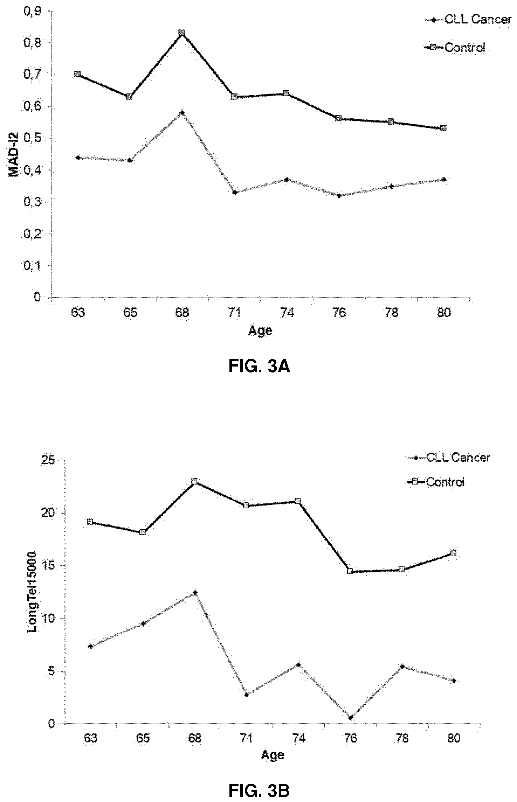

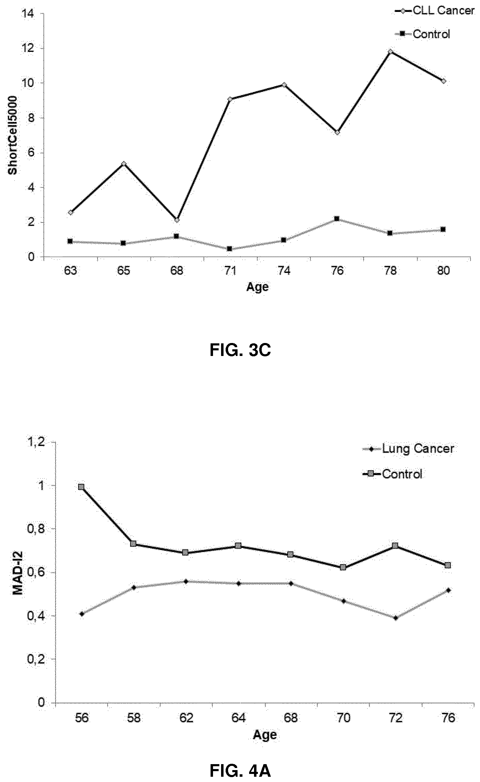

FIG. 3. The TAVs of the invention, such as (**A**) MAD-I2, (**B**) long telomeres > 15,000 pb (LongTel15000) and (**C**) cells having short telomeres < 5,000 pb (ShortCell5000), analyzed in biological sample from subjects having CLL cancer (positive biopsy) and from healthy control subject across the different age-ranges. Diamond shaped marked line represents patients diagnosed with Lung cancer. Square shaped marked line represents healthy controls matched by age.

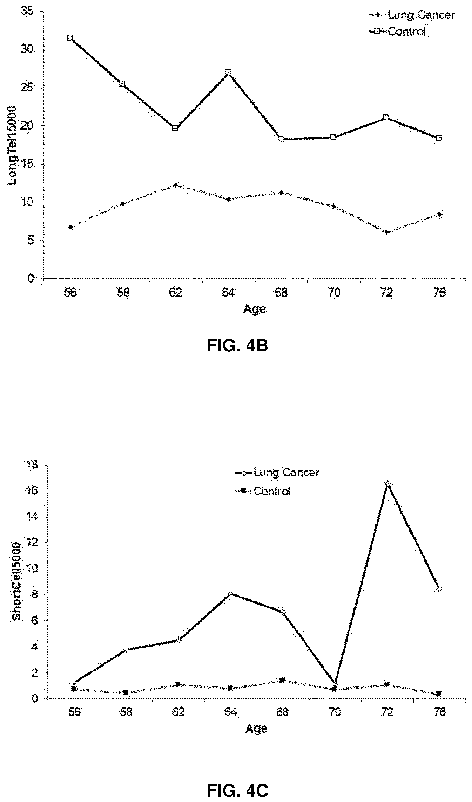

FIG. 4. The TAVs of the invention, such as (**A**) MAD-I2, (**B**) long telomeres > 15,000 pb (LongTel15000) and (**C**) cells having short telomeres < 5,000 pb (ShortCell5000), analyzed in biological sample from subjects having lung cancer and from healthy control subjects across the different age-ranges. Diamond shaped marked line represents patients diagnosed with lung cancer. Square shaped marked line represents healthy controls matched by age.

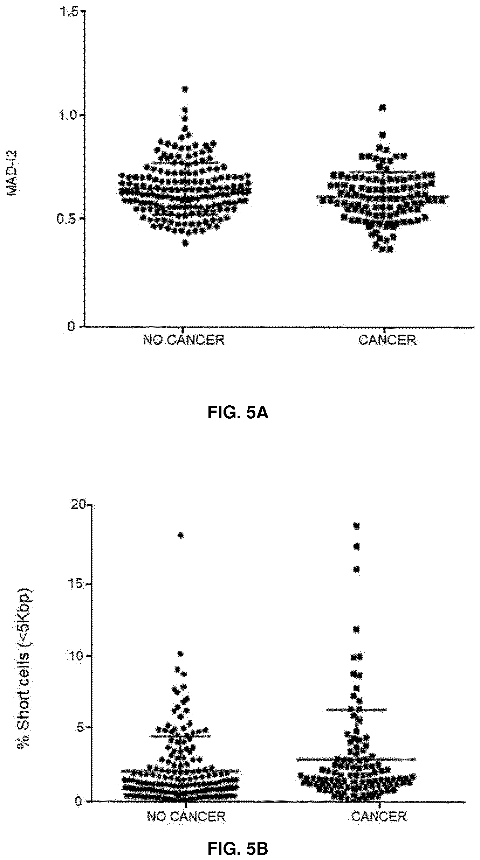

**FIG. 5.** The TAVs of the invention, such as (**A**) MAD-I2 and (**B**) the percentage of cell having short telomeres (<5 Kbp), analyzed in biological sample from subjects having cancer and from healthy control.

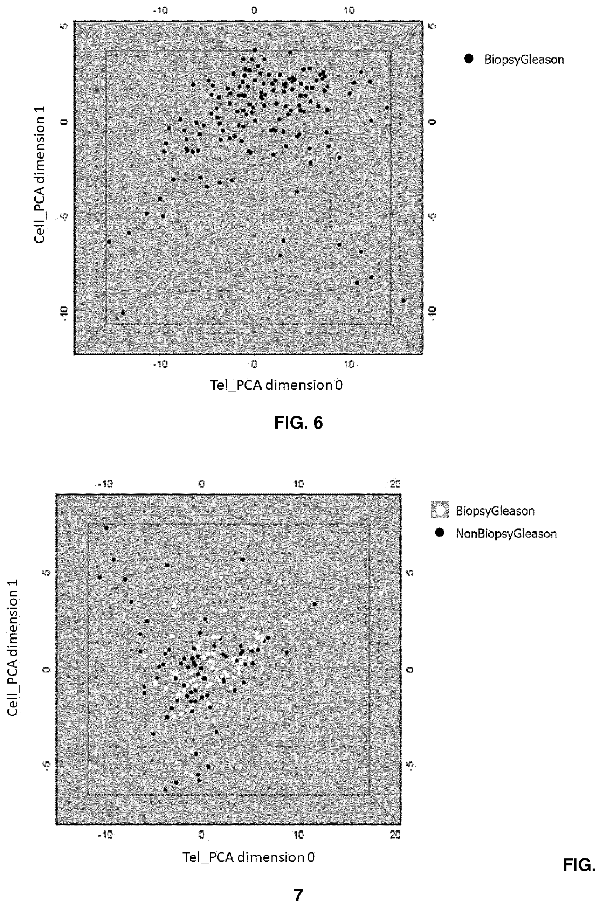

**FIG. 6.** Principal Component Analysis comprising data from the patients (n=401) using the TAVs of the invention. X- axis shows the PCA of the telomeres (Tel_PCA dimension 0); Y-axis shows the PCA of cellular data (Cell_PCA dimension 1) and the Z-axis shows the MAD-I2.

**FIG. 7.** PCA using TAVs of the invention (n= 401). White dots representing patients that need to have a confirmatory biopsy and black dots representing patients that do not require a confirmatory biopsy. X- axis shows the Tel_PCA dimension 0; Y-axis shows the Cell_PCA dimension 1 and the Z-axis shows the MAD-I2.

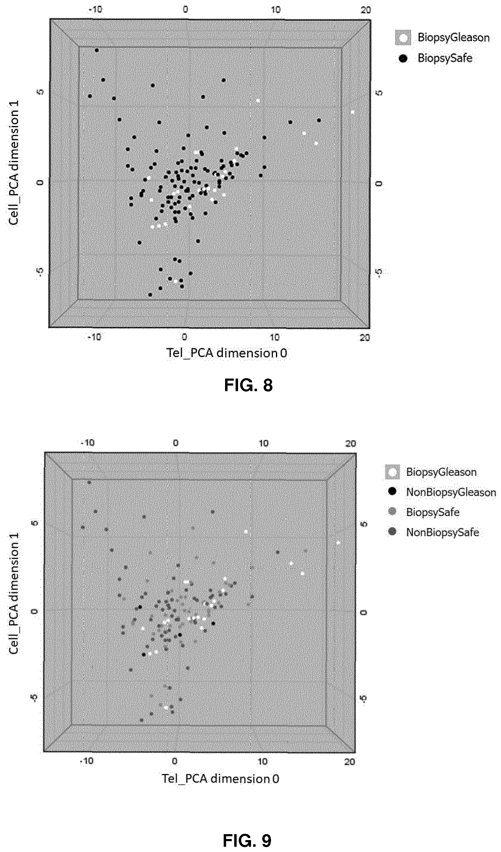

**FIG. 8.** PCA using TAVs of the invention (n= 401). Black dots represent patients with no significant cancer found after biopsy and white dots represent patients with significant cancer diagnosed after biopsy. X- axis shows the Tel_PCA dimension 0; Y-axis shows the Cell_PCA dimension 1 and the Z-axis shows the MAD-I2

**FIG. 9.** PCA using TAVs of the invention (n= 401). White dots represent patients with significant cancer found after biopsy by the method of the invention based on the TAVs of the invention, wherein the data obtained indicates that they should have a biopsy (true positives); light grey dots represent patients that the TAVs obtained by the method of the invention indicate that they should have a biopsy yet it was not necessary (false positives); dark grey dots represent patients that the TAVs obtained by the method of the invention indicate that they did not have a biopsy and indeed they did not need one (true negatives) and black dots represents patients with cancer diagnosed after biopsy wherein the TAVs obtained by the method of the invention indicate that they should not have a biopsy (false negatives). X- axis shows the Tel_PCA dimension 0; Y-axis shows the Cell_PCA dimension 1 and the Z-axis shows the MAD-I2.

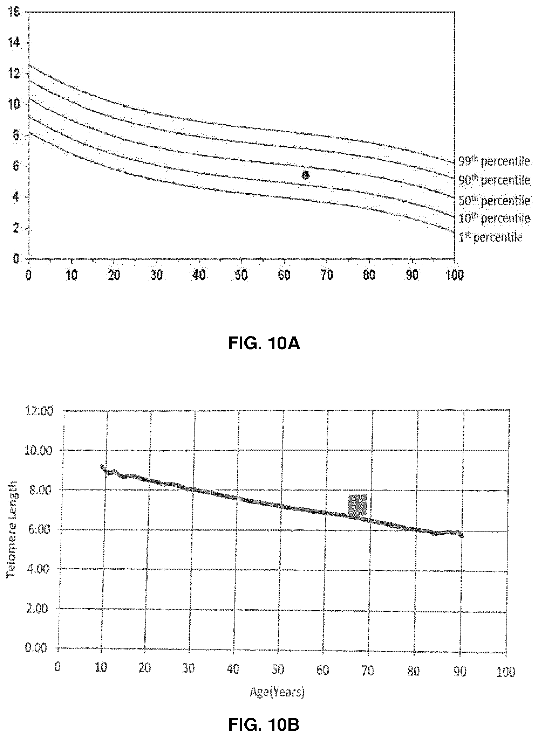

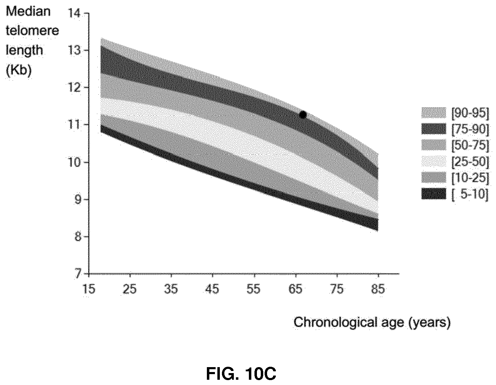

**FIG. 10.** Normograms shown the telomere length obtained by qPCR (**A**), Flow-FISH (**B**) and by the *in vitro* method of the invention which show the data obtained by the analysis of the TAV: median telomere length (Kb) (**C**).

## EXAMPLES

All references cited herein are incorporated by reference in their entirety. The following examples are provided for reference and are not limiting. The present invention is also described and demonstrated by way of the following examples. However, the use of these and other examples anywhere in the specification is illustrative only and in no way limits the scope and meaning of the invention or of any exemplified term. Likewise, the invention is not limited to any particular preferred embodiments described here. Indeed, many modifications and variations of the invention may be apparent to those skilled in the art upon reading this specification, and such variations can be made without departing from the invention in spirit or in scope. The invention is therefore to be limited only by the terms of the appended claims along with the full scope of equivalents to which those claims are entitled.

**Example 1. TAVs constructed from population studies of healthy individuals (Normogram).**

**Method**

Blood samples from healthy individuals were collected voluntarily under ethically approved clinical study protocols and health-related data were also gathered to ensure absence of disease. The peripheral blood mononuclear cells were isolated from the blood samples, counted and stored frozen until used. To perform the method cells were plated on 384-well microtiters plates to perform HT-Q-FISH (Canela A. et al., Proc Natl Acad Sci USA. 2007 Mar 27;104(13):5300-5).

Immortalized, human cell lines were used to stablish a standard curve where individual telomere intensities are interpolated to calculate the TAVs of the invention in healthy individuals from 0 to 93 years of age. The immortalized human lymphoblast cell lines used included commercially available lines from the American Tissue Culture Collection (ATCC #CCL-159, ATCC#CCL-119, ATCC#ccl-86 and ATCC#crl-8286) and from the European Collection of Authenticated Cell Cultures (ECACC #91071212 and ECACC#93012805) as well as from the Life Length collection (LL#125).

Image Acquisition and Analysis. Quantitative image acquisition and analysis is performed on a High Content Screening Opera System (Perkin Elmer), using the Acapella software, Version 2.0 (Perkin Elmer). Images are captured, using a 40x 0.95 NA water immersion objective. UV and 488 nm excitation wavelengths are used to detect the DAPI and A488 signals respectively. With constant exposure settings, at least 15 independent images are captured at different positions for each well. Next, the nuclei images are used to define the region of interest for each cell, measuring telomere fluorescence intensity of the A488 image in all of them. The results of intensity for each foci are exported to the Columbus 2.4 software (Perkin Elmer).

Telomere length distribution, median telomere length and other TAVs such as MAD-12, LongTel15000 and ShortCell5000 are calculated using the Plate Analyzer program of Life Length. For quantitative analysis to generate the TAVs and obtain the distribution of these TAVs in a biological sample, Plate Analyzer analysis software developed by Life Length SL (Madrid, Spain) is used. In order to facilitate processing, the analysis can be configured with filters that enable threshold configuration at multiple points during the data acquisition and data integration that determine the quality of the data and whether they must be kept, discarded, or marked as invalid.

The software works on two different dimensions - both intensity and length, with length being derivable at any point from intensity through a configurable interpolation. Both these dimensions can be analysed at the individual telomere level as well as at other levels - including but not limited to a cell, group of cells, well or aliquot. Statistical information about the distribution is obtained at any of these levels.

## Results

Blood samples from healthy subjects from zero to 93 years of age were analysed by the *in vitro* method of the invention to obtain the specific three TAVs. The data was plotted such that the age of the subject was paired with the 50th percentile of the telomere length of the sample. The 50th percentile of the distribution corresponds with the median telomere length of the sample.

A total of 2,652 samples were used to obtain the normogram of the population for the TAVs of the invention, this is MAD-I2 of the telomeric length, percentage of long telomeres (LongTel15000) and percentage of cells having short telomeres (ShortCell5000). For each TAVs resulting from the *in vitro* method of the invention the different percentiles curves were also calculated.

TAVs analysed are age dependent and the method allows the generation of a database to stablish the control or references values to compare test samples.

**Fig. 1A** shows that the TAV MAD-I2 is a variable that decreases with age. In the same sense, **Fig. 1B** shows that the percentage of cell having long telomeres (at least 15,000 pb) (LongTel15000) is a TAV that decreases with age and moreover, **Fig. 1C** shows that the percentage of cells with short telomeres (less than 5,000 pb) (ShortCell5000) increases with age.

## Example 2. The value of the TAVs of the invention are significantly different in solid tumors and haematological cancer when compared to the TAVs value from non-cancer subjects.

## Method

Blood samples from healthy subjects as well as from individuals in the early stages of prostate cancer (PC), lung cancer (LC) and chronic lymphocytic leukaemia (CLL) were collected voluntarily under ethically approved clinical study protocols and clinical-related data were also gathered. The peripheral blood mononuclear cells were isolated from the blood samples, counted and stored frozen until used. To perform the method cells were plated on 384- well microtiters plates to perform HT-Q-FISH (Canela A. et al., Proc Natl Acad Sci USA. 2007 Mar 27;104(13):5300-5).

Cell lines selected from IM9, CEM, C0106, COJ, C0154 RAJI, REH and 1301 are used to stablish a standard curve where individual telomere intensities are interpolated to calculate the TAVs of the invention from healthy subjects and subjects in the early stage of diagnostic for prostate cancer, lung cancer and CLL.

Image Acquisition and Analysis was performed as disclosed in Example 1.

## Results

### Prostate cancer.

TAVs data results to study individuals selected from a group of male subjects that have a prostate specific antigen blood test (PSA) greater than 3 ng/ml were tested for telomere associated variables according to the *in vitro* method of the invention. Other clinical variables recorded were age, digital rectal examination and free-PSA.

All patients with PSA above 3 ng/ml underwent a prostate biopsy to diagnose if the elevated PSA levels were caused by the presence of prostate cancer.

The TAVs analysed by the *in vitro* method of the invention from patients that have a positive biopsy (which have prostate cancer) or have a negative biopsy (which do not have prostate cancer) were observed to be significantly different across the different age-ranges as depicted in **Table 1** and **FIG. 2A-C.**

Table 1. Shows the TAVs of the invention for biopsy positive (prostate cancer) and biopsy negative (non-prostate cancer) patients matched by age.

| Age | Cancer | TAVs | | |
|---|---|---|---|---|
| | | MAD-I2 | Long telomeres (LongTel15000) | Cell having short telomeres (ShortCell5000) |
| 83 | NO | 0.71 | 15.34 | 0.25 |
| 83 | YES | 0.46 | 10.07 | 2.33 |
| | | | | |
| 80 | NO | 0.85 | 23.19 | 0.52 |
| 80 | YES | 0.56 | 14.66 | 1.59 |
| | | | | |
| 77 | NO | 0.64 | 23.58 | 0.84 |
| 77 | YES | 0.57 | 12.34 | 4.35 |
| | | | | |
| 73 | NO | 0.8 | 18.36 | 0.35 |
| 73 | YES | 0.61 | 7.7 | 0.58 |
| | | | | |
| 70 | NO | 0.69 | 14.12 | 0.75 |
| 70 | YES | 0.48 | 9.69 | 1.62 |
| | | | | |
| 67 | NO | 0.87 | 25.17 | 0.36 |
| 67 | YES | 0.51 | 13.1 | 5.7 |
| | | | | |
| 62 | NO | 0.64 | 25.52 | 0.27 |
| 62 | YES | 0.49 | 11.1 | 2.86 |
| | | | | |
| 51 | NO | 0.77 | 26.69 | 0.66 |
| 51 | YES | 0.37 | 6.19 | 15.82 |

The results showed in **Table 1** and **FIG. 2A-C** point out that the percentage of telomeres longer than 15,000 bp is greater in the patients without a cancer **(FIG. 2B).** Moreover, the MAD-I2 of the telomere distribution on each of the samples is lower in cancer patients **(FIG. 2A)** and the percentage of cells having a median telomere below 5,000 bp is greater in subjects with positive prostate cancer biopsies **(FIG. 2C).**

**Chronic Lymphocytic leukaemia (CLL)**

Individuals selected from a group of subjects recently diagnosed with CLL were tested for the TAVs of the invention. Other clinical variables recorded were age, gender, disease's stage and p53 mutation.
All patients diagnosed with CLL were paired with healthy controls matched by age.
As shown in **Table 2** and **FIG. 3A-C,** significant differences were observed for each of the TAVs of the invention in healthy control (no cancer) versus CLL cancer patients. The significant differences are age-related such that for elderly patients (>75 years of age) as compared with younger individuals (>65 years of age), the TAV MAD-I2 is significantly reduced while the TAV Shortcell5000 (percentage of cell having short telomeres with a length less than 5, 000 pb) is significantly increased.

**Table 2.** Shows the TAVs obtained by the *in vitro* method of the invention for CCL patients and healthy control subject matched by age.

| Age | Cancer | MAD-I2 | TAVs | |
| --- | --- | --- | --- | --- |
| | | | Long telomeres (LongTel15000) | Cell having short telomeres (ShortCell5000) |
| 83 | NO | 0.71 | 15.34 | 0.25 |
| 83 | YES | 0.46 | 10.07 | 2.33 |
| | | | | |
| 80 | NO | 0.85 | 23.19 | 0.52 |
| 80 | YES | 0.56 | 14.66 | 1.59 |
| | | | | |
| 77 | NO | 0.64 | 23.58 | 0.84 |
| 77 | YES | 0.57 | 12.34 | 4.35 |
| | | | | |
| 73 | NO | 0.8 | 18.36 | 0.35 |
| 73 | YES | 0.61 | 7.7 | 0.58 |
| | | | | |
| 70 | NO | 0.69 | 14.12 | 0.75 |
| 70 | YES | 0.48 | 9.69 | 1.62 |
| | | | | |
| 67 | NO | 0.87 | 25.17 | 0.36 |
| 67 | YES | 0.51 | 13.1 | 5.7 |
| | | | | |
| 62 | NO | 0.64 | 25.52 | 0.27 |
| 62 | YES | 0.49 | 11.1 | 2.86 |
| | | | | |
| 51 | NO | 0.77 | 26.69 | 0.66 |
| 51 | YES | 0.37 | 6.19 | 15.82 |

The results show that the percentage of telomeres longer than 15,000 bp is significantly greater in the no-cancer patients (**FIG. 3B**), the MAD-I2 is significantly lower in cancer patients (**FIG. 3A**) and the percentage of cells with median telomere length below 5,000 bp is significantly greater in subjects that have been diagnosed with CLL (**FIG. 3C**).

**Lung cancer.**

Individuals selected from a group of subjects with suspected lung cancer were tested for telomere associated variables. Other clinical variables recorded were age, gender, disease stage and tobacco consumption. All patients diagnosed with lung cancer were compared with healthy controls matched by age. Differences were observed for the TAVs of in the invention examined for all ages.

Table 3. The TAVs obtained by the in vitro method of the invention for lung cancer patients and healthy controls matched by age.

| Age | Cancer | TAVs | | |
|---|---|---|---|---|
| | | MAD-I2 | Long telomeres (LongTel15000) | Cell having short telomeres (ShortCell5000) |
| 76 | NO | 0.63 | 18.26 | 0.35 |
| 76 | YES | 0.52 | 8.44 | 8.44 |
| | | | | |
| 72 | NO | 0.72 | 21,00 | 1.04 |
| 72 | YES | 0.39 | 6.04 | 16.54 |
| | | | | |
| 70 | NO | 0.62 | 18.48 | 0.71 |
| 70 | YES | 0.47 | 9.44 | 1.14 |
| | | | | |
| 68 | NO | 0.68 | 18.16 | 1.38 |
| 68 | YES | 0.55 | 11.25 | 6.66 |
| | | | | |
| 64 | NO | 0.72 | 26.9 | 0.77 |
| 64 | YES | 0.55 | 10.46 | 8.11 |
| | | | | |
| 62 | NO | 0.69 | 19.61 | 1.06 |
| 62 | YES | 0.56 | 12.23 | 4.48 |
| | | | | |
| 58 | NO | 0.73 | 25.38 | 0.42 |
| 58 | YES | 0.53 | 9.8 | 3.78 |
| | | | | |
| 56 | NO | 0.99 | 31.45 | 0.73 |
| 56 | YES | 0.41 | 6.83 | 1.25 |

The results obtained show that the percentage of telomeres longer than 15,000 bp is significantly greater in the no-cancer patients (healthy controls) versus lung cancer patients (FIG. 4B). Moreover, the MAD-I2 on each of the samples is significantly lower in lung cancer patients versus non-cancer patients (FIG. 4A) and the percentage of cells with median telomere length below 5,000 bp is significantly greater in subjects that have been diagnosed with lung cancer versus no-cancer patients (healthy controls) (FIG. 4C).

**Example 3. TAVs obtained by the *in vitro* method of the invention for patient triage into confirmatory biopsy following PSA determination >3 ng/ml.**

**Method**

Blood samples from healthy subjects as well as from individuals in the early stage of diagnostic for prostate cancer (PC) were collected voluntarily under ethically approved clinical study protocols and clinical-related data were also gathered. The peripheral blood mononuclear cells were isolated from the blood samples, counted and stored frozen until used. To perform the method cells were plated on 384-well microtiters plates to perform HT-Q-FISH (Canela A. et al., Proc Natl Acad Sci USA. 2007 Mar 27;104(13):5300-5). Image Acquisition and Analysis of the blood samples was performed as in Example 1.

The TAVs data results from a group of 150 male subjects that have a prostate specific antigen blood test (PSA) greater than 3 ng/ml and lower than 10 ng/ml were used in combination with the *in vitro* method of the invention to validate the TAVs obtained by this method. To do this, the clinical data mentioned herein with the TAVs obtained by the *in vitro* method of the invention were used in combination as part of a predictor algorithm (Life Length S.L. Madrid, Spain) to determine if a subject that is suspected of prostate cancer has to be biopsied or not.

To integrate the TAVs of the method of the invention a Principal Component Analysis (PCA) was performed. The variable set to test includes a combination of Principal Component Analysis (PCAs). A total of six combinations were done each of them combining percentile variables, LongTel (percentage of telomeres longer than 15,000 bp) variables, and ShortCell (cells with short telomeres less than 5,000pb) variables - along with the variables MAD-I2, P75-25 (Q3-Q1) and P1-99. A total of five PCA were generated. The principal components for each group are numbered from 0 to 5.

**Table 4.** Shows the distribution of the 150 patients included in this analysis divided in those that were diagnosed with cancer, those deemed cancer negative (no cancer) and within the cancer group those that have a significant cancer (Gleason) and those that had a non-significant cancer (No Gleason).

|  | *Cancer* | *No Cancer* | *Total* |
|---|---|---|---|
| *No Gleason* | 36 | 85 | 121 |
| *Gleason* | 29 | 0 | 29 |
| *Total* | 65 | 85 | 150 |

**Results**

When patients were grouped into those diagnosed with cancer and cancer free (no-cancer), the TAVs MAD-I2 and cell having short telomeres less than 5, 000 pb (ShortCell5000) analysed by the *in vitro* method of the invention showed differences in their distribution (**FIG. 5A-B**). The data showed in **FIG. 5** are not segmentation by age.

The accuracy for the TAVs of the invention, alone (MAD-I2 or LongTel15000 or ShortCell5000) or in any combinations thereof (MAD-I2 or MAD-I2 and LongTel15000 or MAD-I2 and LongTel15000 and ShortCell5000) was calculated by the *in vitro* method of the invention and the results obtained show an increase in the percentage for cancer accuracy prediction as more TAVs of the invention are taken into account (**Table 5**).

**Table 5.** Accuracy for the TAVs of the invention alone or in combinations thereof.

| *TAVs* | *Accuracy* |
|---|---|
| MAD-I2 | 52% |
| MAD-I2+ShortCell5000 | 54% |
| MAD-I2+ShortCell5000+ShortTel15000 | 55% |

Once PCA were created a two-tailed t-test assuming unequal variances is performed on each of the aforementioned variables.

**Table 6.** List of variables analysed by Principal Component Analysis of the data to establish whether they were significantly different (p< 0.05) between cancer and no-cancer groups of patients.

| Variable | p-value |
|---|---|
| Perc_PCA dimension 0 | 8.00E-05 |
| Perc_PCA dimension 1 | 0.52 |
| Perc_PCA dimension 2 | 0.85 |
| Perc_PCA dimension 3 | 0.3 |
| Perc_PCA dimension 4 | 0.03 |
| Perc_PCA dimension 5 | 0.16 |
| Tel_PCA dimension 0 | 7.00E-05 |

(continued)

| Variable | p-value |
|---|---|
| Tel_PCA dimension 1 | 0.78 |
| Tel_PCA dimension 2 | 0.5 |
| Tel_PCA dimension 3 | 0.8 |
| Tel_PCA dimension 4 | 0.78 |
| Tel_PCA dimension 5 | 0.38 |
| Cell_PCA dimension 0 | 2.00E-04 |
| Cell_PCA dimension 1 | 0.32 |
| Cell_PCA dimension 2 | 0.42 |
| Cell_PCA dimension 3 | 0.77 |
| Cell_PCA dimension 4 | 0.83 |
| Cell_PCA dimension 5 | 0.17 |
| MAD-I2 | 8.00E-05 |
| Percentil 1-99 | 8.00E-04 |
| Percentil 25-75 | 0.05 |

The result shows in **Table** 6 indicates that, MAD-I2 is an order of magnitude above the percentiles P1-99 and the percentile P25-75 is not statistically significant. Each of the PCAs dimension refers to dimension reduction or the process of reducing the number of random variables under consideration. Dimensions zero are statistically significant while mostly the other dimensions are not, with the only exception of Percentile PCA for the dimension 4, which is statistically significant. This data points towards an uneven distribution of information among the multiple PCs so that the vast majority of the information for each group of variables is contained in the initial dimension, which shows significant differences between cancer/no cancer groups. This is in line with the high amount of statistically significant variables that are processed to generate the PCAs, a total of 209 out of which 186 are significant.

The **FIG. 6** shows all the patients included in the Principal Component Analysis using TAVs.

**FIG. 7** shows all patients included in the PCA using TAVs colour-coded according to their classification by the model into white dots representing patients that need to have a confirmatory biopsy and black dots representing patients that do not require a confirmatory biopsy.

**FIG.** 8 shows all patients included in the PCA using TAVs colour-coded according to their biopsy result as well as the significance or not of the diagnosed cancer. Black dots represent patients with no significant cancer found after biopsy, white dots represent patients with significant cancer diagnosed after biopsy.

**FIG. 9** shows all patients included in the PCA using TAVs colour-coded according to the prediction of the model generated using the clinical data of their biopsy result. White dots represent patients with significant cancer found after biopsy that the model based on TAVs indicates they should have a biopsy (true positives), light grey dots represent patients that the model found should have a biopsy yet it was not necessary (false positives), dark grey dots represent patients that the model indicates did not have a biopsy and indeed they did not needed (true negatives) and black dots represents patients with cancer diagnosed after biopsy that the model indicates they should not have a biopsy (false negatives).

**Example 4. The method to determine TAVs and not other telomere length determination methods can inform of median average deviation, percentage of long telomeres and percentages of cells with short telomeres.**

**Method**

In order to compare the in vitro method of the invention versus different methods known in the art for the assaying of TAVs (i.e. qPCR and Flow FISH) a biological sample from the same subject was analysed by qPCR, Flow FISH and by the *in vitro* method of the invention. qPCR was performed in a commercial laboratory (Spectra Cell Laboratories, Houston, Texas). Flow-FISH was performed in a commercial laboratory (Repeat Diagnostic, Vancouver, Canada) and HT-QFISH was performed at Life Length SL. (Madrid, Spain).

## Results

The data related to telomeres such as the average length of the telomeres in the biological sample or the length of particular cell type was obtained. The same biological sample was analysed by the *in vitro* method of the invention to determined TAVs and it is the only method that can determine variables associated with health and disease status such as MAD-I2, LongTel15000 and ShortCell5000.

**Table 7.** Shows data from a blood sample processed by Flow-FISH. MTL: Patient Median Telomer Length; MTLN: Normal MTL at age (50th percentile) and INT: Telomere length interpretation.

| Lymphocytes | | |
|---|---|---|
| MTL (kb) | MTLN (kb) | INT (kb) |
| 5.4 | 6.0 | N |
| Granulocytes | | |
| MTL (kb) | MTLN (kb) | INT (kb) |
| 6.4 | 7.8 | N |

The associated results (**Table 7**) can determine the sample median telomere length -median distribution of the telomere length calculated per cell and can specify if the cell are lymphocytes or granulocytes. However, the method cannot calculate the MAD-I2 or the percentage of long telomeres in the sample.

**Table 8.** Shows data from a blood sample processed by qPCR.

| Patient Telomere Score | 7.26% |
|---|---|
| Percentile relative to patient age and population | 73% |

The associated results (**Table 8**) can determine the sample average telomere length relative to a standard. qPCR cannot generate values on the TAVs of the invention, such as MAD-I2, percentage of long telomeres and percentage of cells having short telomeres.

**Table 9.** Shows data from a blood sample processed by the in vitro method of the invention (HT-Q-FISH).

| TAVs | Base pair (bp) |
|---|---|
| Average length (bp) | 13130 |
| Median length (pb) | 11296 |
| % Long Telomeres [LongTelPercent (15000pb)] | 72.5 |
| % Cell having short telomeres [ShortCellPercent (5000pb)] | 0.25 |
| MAD-I2 | 0.71 |

The associated results obtained by the *in vitro* method of the invention (**Table 9**) can determine the sample median and average telomere length in absolute base pair units. In addition, data on the TAVs such as MAD-I2, Longtel15000 and Shortcell5000 can also be obtained.

FIG. 10 shows different normograms obtained by qPCR (**FIG. 10A**), Flow-FISH (**FIG. 10B**) and HT-Q-FISH (**FIG. 10C**) based on average or median telomere length calculated over a group of healthy subjects of different ages. The slopes and the distribution of the data is different for each method.

## Claims

1. Method *in vitro* for measuring Telomere Associated Variables (TAVs) in an isolated biological sample of a subject, wherein the method comprises the following steps:

    a) assaying, in the isolated biological sample, the TAVs selecting from the list consisting of: absolute deviation of the median (MAD 12) of the telomeric length, percentage of long telomeres and percentage of cells having short telomeres,
b) comparing the TAVs obtained in step a) with the TAVs obtained in a reference sample, and
c) identifying a significant difference between the TAVs comparison of step b),

wherein a significant difference is indicative of: a measure of health; a risk of a pathological condition; a telomeric disease or drug responsiveness.

2. The method *in vitro* according to claim 1, wherein the MAD I2 of the telomeric length is determined by the formula:

$$MAD = median(|m - \tilde{m}|)$$

wherein

m is the set of fluorescence intensities corresponding to the telomeres in the sample $M_t$ and $\tilde{m}$ is the median of m, m = {$t_1, t_2, ..., t_n$};
$M_t$ = {$T_1, T_2, ..., T_n$} is the complete set of telomeres measured in the isolate biological sample; and
$t_i$ ({$t_1, t_2, ..., t_n$}) is the fluorescence intensity of the telomere $T_i$ ({$T_1, T_2, ..., T_n$}).

3. The method *in vitro* according to claim 1, wherein the percentage of long telomeres is determined by the formula:

$$TL_u = 100 - TS_u$$

wherein the percentage of short telomeres $TS_u$ in the isolated biological sample *Mt*, for a threshold length *u* is determined as:

$$TS_u = 100 * \frac{\sum_{i=1}^{n}[t_i < u]}{n}$$

wherein

$M_t$ = {$T_1, T_2 ... T_n$} is the complete set of the telomeres measured in the isolated biological sample $M_t$,
*n* is the number of telomeres in $M_t$, and,
$t_i$ ({$t_1, t_2, ..., t_n$}) is the telomeric length of the telomere $T_i$ ({$T_1, T_2, ..., T_n$}).

4. The method *in vitro* according to claim 1, wherein the percentage of cells having short telomeres ($CS_u$) in the isolated biological sample M for a threshold length *u* is determined by the formula:

$$CS_u = 100 * \frac{\sum_{r=1}^{n}[c_r < u]}{n}$$

wherein:

$c_r$ is the length of $C_r \in M_c$ calculated as: $c_r = avg(t_{ri})$;
*n* is the number of cells in $M_c$,
$t_{ri}$ is the telomeric length of the telomere $T_{ri}$;
$K_r$ = {$Tr_1, Tr_2, ..., T_{rn}$} is the set of telomeres analyzed in a cell $C_r$; and
$M_c$ = {$C_{r1}, C_{r2}, ..., C_m$} is the complete set of cells in the isolated biological sample M.

5. The method *in vitro* according to any of claims 3 or 4, wherein the threshold length *u* ranges from 500 to 40,000 pb in increments of 100 pb.

6. The method *in vitro* according to any of claims 1 to 5 wherein further comprises in step a) the analysis of at least one of the following TAVs selected from the list consisting of: the percentiles 1th to 99th of the telomere distribution, the interquartile Q3-Q1 of the telomere distribution, the nuclear size distribution of the cells, or any combinations thereof.

7. The method *in vitro* according to any of claims 1 to 6, wherein the TAVs are determined by HT Q-FISH.

8. The method *in vitro* according to any of claims 1 to 7, wherein further comprises the measurement of the telomerase activity in the isolated biological sample.

9. The method *in vitro* according to claim 8 wherein the telomerase activity is determined by Q-TRAP.

10. The method *in vitro* according to any of claims 1 to 9 wherein the isolated biological sample is selected from: cells, tissues and/or biological fluid.

11. The method *in vitro* according to any of claims 1 to 10 wherein the isolated biological sample is selected from the list consisting of: plasma, serum, blood, lymphatic fluid, cerebrospinal fluid, synovial fluid, urine, saliva, mucous, phlegm and sputum, preferably plasma, serum or blood.

12. The method *in vitro* according to any of the claim 1 to 11 wherein the pathological condition is a telomeric-associated disease.

13. The method *in vitro* according to any of the claim 1 to 12 wherein the telomeric-associated disease is selected from the list consisting of: cancer, cardiovascular disease, dyskeratosis congenita, pulmonary fibrosis, aplastic anaemia, interstitial pneumonia, diabetes, infertility, haematological diseases, central nervous system diseases, liver diseases and metabolic diseases.

FIG. 1A

FIG. 1B

FIG. 1C

FIG. 2A

FIG. 2B

FIG. 2C

FIG. 3A

FIG. 3B

FIG. 3C

FIG. 4A

FIG. 4B

FIG. 4C

FIG. 5A

FIG. 5B

**FIG. 6**

**FIG. 7**

FIG. 8

FIG. 9

**FIG. 10A**

**FIG. 10B**

FIG. 10C

Europäisches
Patentamt

European
Patent Office

Office européen
des brevets

# EUROPEAN SEARCH REPORT

Application Number

EP 18 38 2992

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GERALDINE AUBERT ET AL: "Telomere length measurementCaveats and a critical assessment of the available technologies and tools", MUTATION RESEARCH, ELSEVIER, AMSTERDAM, NL, vol. 730, no. 1, 6 April 2011 (2011-04-06), pages 59-67, XP028436152, ISSN: 0027-5107, DOI: 10.1016/J.MRFMMM.2011.04.003 [retrieved on 2011-04-13] * page 61 * | 1,2,5-13 | INV. C12Q1/6841 G16B5/00 |
| X | WO 2014/085632 A1 (GERON CORP [US]) 5 June 2014 (2014-06-05) * claims 1-3 * | 3,4 | |
| X | WO 2013/102116 A1 (TELOME HEALTH INC [US]) 4 July 2013 (2013-07-04) * claims 60,68 * | 3,4 | |
| X | US 8 084 203 B2 (FLORES HERNANDEZ IGNACIO [ES]; CANELA RODRIGUEZ ANDRES [US] ET AL.) 27 December 2011 (2011-12-27) * examples 4,5 * | 3,4 | TECHNICAL FIELDS SEARCHED (IPC)  G06F C12Q G16B |
| A | JEANETTE E ECKEL-PASSOW ET AL: "Software comparison for evaluating genomic copy number variation for Affymetrix 6.0 SNP array platform", BMC BIOINFORMATICS, BIOMED CENTRAL, LONDON, GB, vol. 12, no. 1, 31 May 2011 (2011-05-31), page 220, XP021102541, ISSN: 1471-2105, DOI: 10.1186/1471-2105-12-220 * figure 1 * | 1 | |

The present search report has been drawn up for all claims

| Place of search | Date of completion of the search | Examiner |
|---|---|---|
| The Hague | 26 April 2019 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 38 2992

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2019

| Patent document cited in search report | | Publication date | Patent family member(s) | | | Publication date |
|---|---|---|---|---|---|---|
| WO 2014085632 | A1 | 05-06-2014 | AU | 2013352105 | A1 | 18-06-2015 |
| | | | BR | 112015012507 | A2 | 11-07-2017 |
| | | | CA | 2892445 | A1 | 05-06-2014 |
| | | | CN | 104937110 | A | 23-09-2015 |
| | | | DK | 2925889 | T3 | 02-01-2019 |
| | | | EP | 2925889 | A1 | 07-10-2015 |
| | | | ES | 2703914 | T3 | 13-03-2019 |
| | | | HK | 1215874 | A1 | 23-09-2016 |
| | | | HR | P20181991 | T1 | 08-03-2019 |
| | | | JP | 6359028 | B2 | 18-07-2018 |
| | | | JP | 2016501523 | A | 21-01-2016 |
| | | | JP | 2018099127 | A | 28-06-2018 |
| | | | KR | 20150091491 | A | 11-08-2015 |
| | | | LT | 2925889 | T | 27-12-2018 |
| | | | PL | 2925889 | T3 | 28-02-2019 |
| | | | SG | 11201504209Y | A | 29-06-2015 |
| | | | SI | 2925889 | T1 | 31-01-2019 |
| | | | WO | 2014085632 | A1 | 05-06-2014 |
| WO 2013102116 | A1 | 04-07-2013 | AU | 2012362210 | A1 | 21-08-2014 |
| | | | CN | 104105798 | A | 15-10-2014 |
| | | | CN | 105662482 | A | 15-06-2016 |
| | | | EP | 2798091 | A1 | 05-11-2014 |
| | | | HK | 1202593 | A1 | 02-10-2015 |
| | | | TW | 201343919 | A | 01-11-2013 |
| | | | US | 2014370505 | A1 | 18-12-2014 |
| | | | US | 2017023451 | A1 | 26-01-2017 |
| | | | WO | 2013102116 | A1 | 04-07-2013 |
| US 8084203 | B2 | 27-12-2011 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HEMANN, M.T. et al.** *Cell,* 2001, vol. 107 (1), 67-77
- **VERA E. et al.** *Cell Rep.,* 25 October 2012, vol. 2 (4), 732-7
- **CANELA A. et al.** *Proc Natl Acad Sci USA.,* 27 March 2007, vol. 104 (13), 5300-5